Europäisches Patentamt

European Patent Office

Office européen des brevets

(11) Publication number: **0 146 143**
**B1**

(12) **EUROPEAN PATENT SPECIFICATION**

(45) Date of publication of patent specification: **19.09.90**

(51) Int. Cl.⁵: **C 12 M 1/16**

(21) Application number: **84115844.7**

(22) Date of filing: **19.12.84**

(54) Simplified detection implement.

(30) Priority: **20.12.83 JP 240653/83**

(43) Date of publication of application:
**26.06.85 Bulletin 85/26**

(45) Publication of the grant of the patent:
**19.09.90 Bulletin 90/38**

(84) Designated Contracting States:
**DE FR GB NL**

(56) References cited:
**GB-A-2 031 583**
**US-A-4 055 394**

(73) Proprietor: **Showa Yakuhin Kako Co., Ltd.**
**No. 16-5, 1-chome, Kyobashi**
**Chuo-ku Tokyo (JP)**

(73) Proprietor: **NITTO DENKO CORPORATION**
**1-2, Shimohozumi 1-chome Ibaraki-shi**
**Osaka (JP)**

(72) Inventor: **Nagase, Moriharu**
**4-11-15, Kokubunjidai**
**Ebina-shi Kanagawa-ken (JP)**
Inventor: **Shibuya, Mutsumi**
**Kikuna Height 3-106 No. 316-6 Mamedo-cho**
**Kouhoku-ku Yokohama-shi Kanagawa-ken (JP)**
Inventor: **Asami, Kuniaki**
**Arukasaano Kugahara 202 1-16-10, Minami**
**Kugahara**
**Ohtaku Tokyo (JP)**
Inventor: **Matsumoto, Katsuo**
**1-1-1, Shironishi**
**Furukawa-shi Miyagi-ken (JP)**
Inventor: **Teranishi, Hikaru**
**28-30, Ninokamae Iwadeyama-machi**
**Tamatsukuri-gun Miyagi-ken (JP)**
Inventor: **Saito, Sukeo Asahi Plaza Hodogaya**
**101**
**222-190, Fujitsuka-cho Hodogaya-ku**
**Yokohama-shi Kanagawa-ken (JP)**

Courier Press, Leamington Spa, England.

**EP  0 146 143  B1**

(74) Representative: **Vossius & Partner
Siebertstrasse 4 P.O. Box 86 07 67
D-8000 München 86 (DE)**

## Description

This invention relates to a simplified detection implement for testing biological or chemical substances, e.g. for performing medical, pharmaceutical, microbiological, enzymological or analytical tests.

The various tests mentioned above include medical and physical check-ups, clinical tests of patients and chemical analyses of food, pharmaceutical, mineral, animal and plant samples either after collection and storage or right on the spot. Said tests are based on chemical reactions, the reactivity of which is generally higher under application of heat. The required reaction temperature varies from around 20°C, 35°C, 80°C to boiling point.

It is of course desirable for the reaction to proceed at the lowest temperature possible. Attempts have been made to achieve this by modifying the reaction system, reagent, or by adding reaction accelerators or by combining some detection devices. In most cases, a speedy test is possible without side reactions or deterioration in the sensitivity of the reaction when heat of around 35°C is applied. Ideally, all materials should be handled using the same implements, as should all measures, such as collecting, storing, reacting, testing, and preserving the samples and test results be performed with the same implements. Many implements have already been devised with this in mind and put into practical use. Apart from offering other advantages, the existing implements are simple, accurate and have small cubic volume. These features are also desirable from the economical point of view. Also, in medical chemistry it is especially important for the test to be safe both for the patient and the person performing it.

Description of the Invention

The detection implement of this invention is a further improvement of the prior art detection implement in which the porous small substance is soaked in advance with all or a portion of the test reactants. Furthermore, said small material adheres in dry state to a highmolecular film that has a sticky surface. Said sticky surface is protected by a protection sheet, into which an opening is cut in advance. As said opening is made a little bigger than the small substance, said small substance is exposed through this opening. The adhesive surface of the film also serves the purpose of adhering to a heated body or heating apparatus, whereby it receives the reaction temperature.

Brief Description of the Drawings

The attached drawings illustrate the detection implement of this invention.

Fig. 1A is a cross-sectional view;

Fig. 1B is a plane view;

Fig. 2 shows the state just before the detection implement is applied to the heating apparatus with the middle section of the adhesive sheet folded and laid over the other portion after the protection sheet is peeled off from the detection implement in order to render the detection implement ready for testing;

Fig. 3 shows the serially linked type of the detection implement;

Figs. 4 and 5 show another shape (square shape) which the detection implement of this invention may have;

Fig. 5 shows examples of the serially linked type;

Figs. 6-8 show the protection sheet 2 and adhesive sheet 1 as having the same shape and size;

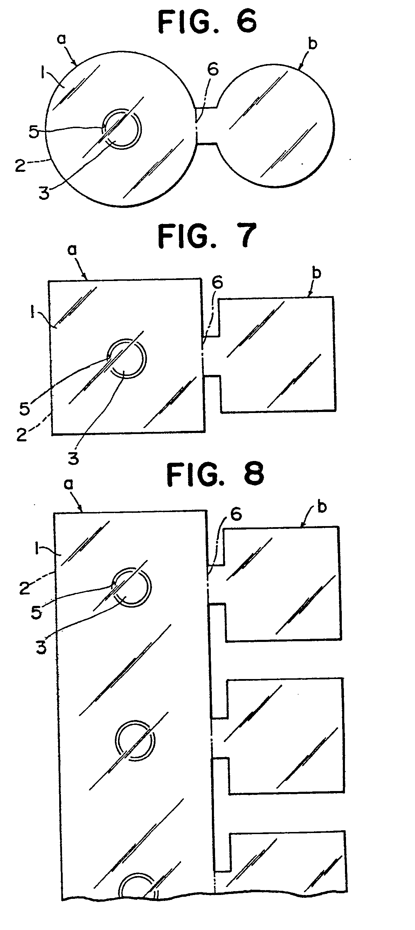

Fig. 6 shows the round shape, Fig. 7 the square shape;

Fig. 8 the serially linked type;

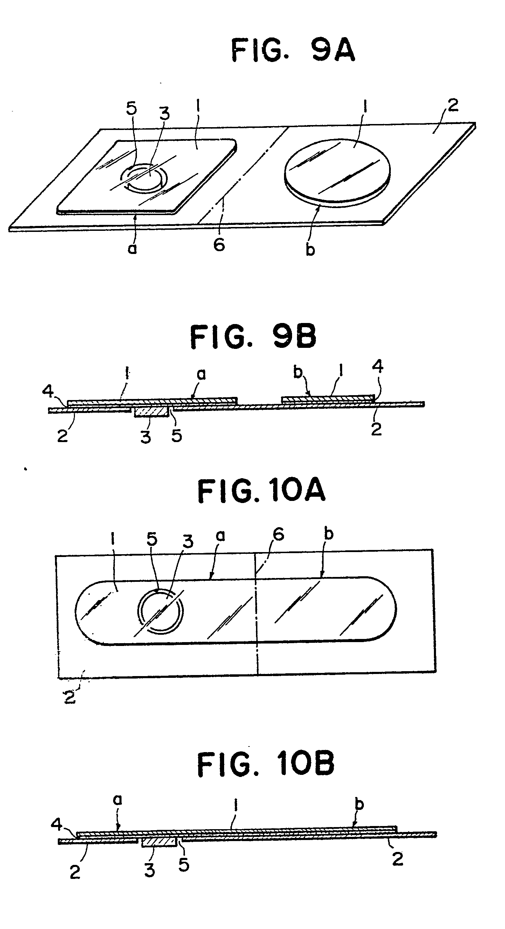

Figs. 9A, 9B, 10A and 10B show other embodiments;

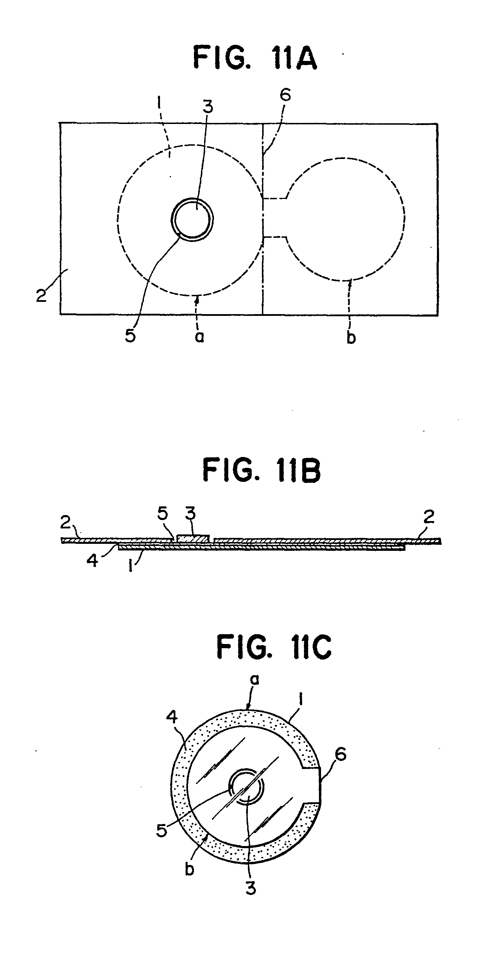

Fig. 11A, 11B and 11C show the basic shapes of the detection implement of this invention and, respectively, represent the plane view, cross-sectional view and the state just before application to a heated body or heating apparatus.

The detection implement of this invention is composed of an adhesive sheet 1 which can be peeled off, a protection sheet 2 and a small test material 3 which is used for detecting the substance to be tested. Said three portions are superimposed upon one other forming a composite structure. Said three portions can all be transparent, some or all may be opaque or some or all of them may be provided with a transparent or opaque printing. The inner surface of the adhesive sheet 1 is partly or wholly covered with the adhesion material 4 and a small test material 3 is affixed close to the center or a little to the left or right side of the center position. The protruding portion around the rim of area "a" of said adhered surface will be the part to stick to the heating apparatus or heated body.

The protection sheet 2 is a thin sheet that is large enough to cover the adhesion material layer 4 of the adhesive sheet 1 or slightly larger; corresponding in shape to the small test material 3, a somewhat larger opening cut or pierced into the separation sheet 2 allows the small test material 3 to be exposed through it. The adhesive sheet 1 is generally transparent. When said sheet 1, to which the small test material 3 adheres is folded at the folding line 6, the surface of the adhesion material layer 4 becomes the inner surface and sticks to and covers the small test material 3. The adhering sheet covering the small test material 3 is smaller in shape than said adhesive sheet, so that the peripheral part of area "a" of the adhesive sheet 1 to which the small test material 3 adheres is exposed. This exposed region will stick to the heated body or heating apparatus.

The small test material 3 is an inorganic or organic porous substance. Conveniently, it is a small piece of paper. The small test material is generally soaked in advance with the test reagent but depending on the purpose of use, it does not have to be soaked in advance.

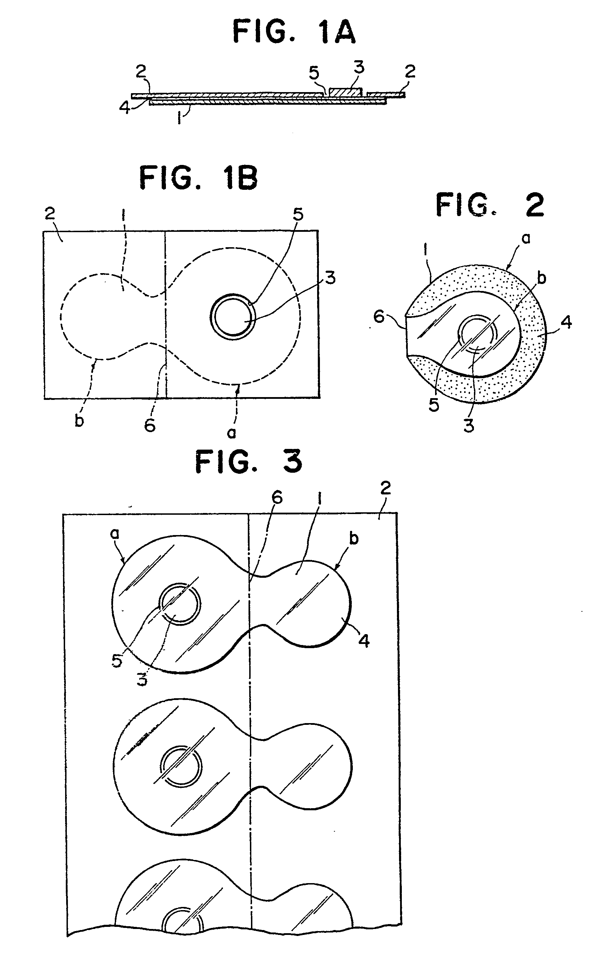

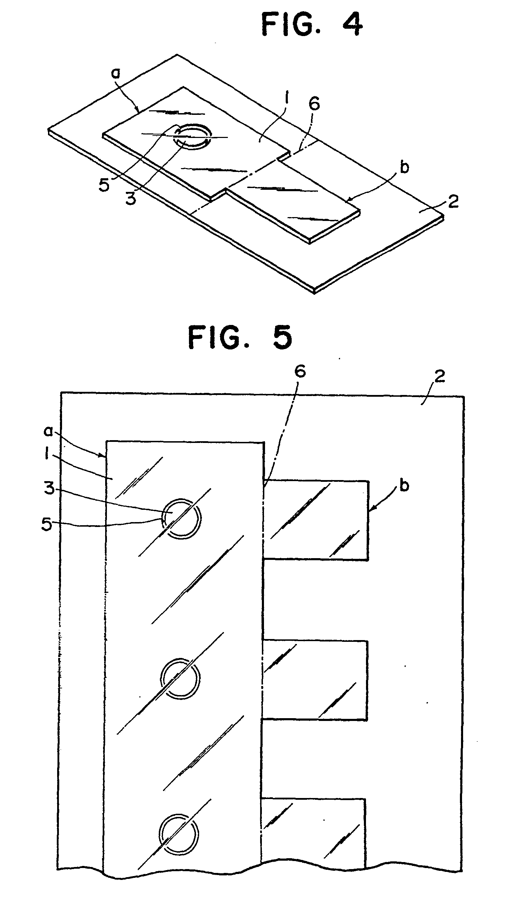

The adhesive sheet 1, illustrated in Figs. 1-3, has a horseshoe, scallop, inverted jar or balloon shape, while the adhesive sheets 1 in Figs. 4-5 show a rectangular shape and when folded will have a square shape. Thus, the selection of the shape of the adhesive sheet 1 is optional. The shape of the areas "a" and "b" is also optional, as they could have a round, oval or polygon shape or combinations of them.

The detection implement of this invention is sold and used in one shape only or in many shapes. Where the implement is given many shapes, these may well be serially linked (Figs. 3, 5 and 8), which is useful in group examination.

According to an embodiment of this invention, the adhesive sheet 1 is a thin, soft film of polyvinyl chloride, polyvinylidene chloride, polyethelene or polypropylene, etc.. Where the adhesive is a film, the "a" area to the right and the "b" area to the left of the folding line (see Fig. 1B) may or may not be made from the same sheet. If said areas are not made from same sheet, the quality and/or thickness of the film may differ. The film of area "a" may be of firm and stiff quality, e.g. rigid polyvinyl chloride, polyester, or polyethylene terephtalate. In this case the "b" area should be made from a soft and thin film and should moreover be a little smaller than the "a" area. The adhesion material 4 is applied on the surfaces of areas "a" and "b" either wholly, as dots or in lines. In some cases, the components and the applied shape of the adhesive may differ.

The protection sheet 2 is ordinarily made from paper coated with a silicone film on one side. The surface of this side is pressed against the adhesion material layer 4 to cover its entire area and to protect it.

The protection sheet has an opening to expose the small test material 3. Said small test material 3 is fixed to area "a" and does not come into contact with the protection sheet.

In this invention, it is very important that the protection sheet 2 does not come into contact with the small test material 3. When the small test material 3 is to be inoculated with a sample taken from the subject to be tested, i.e. a chemical reagent, an animal, plant or mineral liquid, a culture liquid of bacteria, saliva, milk, etc., inoculation can be performed even without the protection sheet being peeled off. In this case, the adhesion material layer 4 is not exposed, which is desirable. Also, if there are no openings in the protection sheet 2, in other words, if the composite structure of the adhesion material layer 4, the protection sheet 2 and the small test material 3 is in sandwich form, a trace amount of the component evaporating from said adhesion material might have an adverse effect on the composition of the reagent. Such are, however, not encountered in this invention.

The detection implement of this invention is manufactured as follows:

The raw materials of both the adhesive sheet 1 and protection sheet 2 are long rolls of film, which are supplied separately to the processing machine. Here the openings, into which the small test material 3 is to be placed, are punctured into the protection sheet at the predetermined positions and at regular intervals. The protection sheet with the openings is then applied and affixed to the adhesive sheet 1. As a result, the adhesion material 4 is exposed through the opening of said protection sheet 2 so that the small test material 3 can adhere and become affixed to said exposed adhesive area. As the detection implement of this invention only requires that a piece of test material be placed into the aforementioned protection sheet, production on an industrial scale is simple and efficient.

The simplified detection implement thus obtained, can, for instance, be airtightly packaged in a metal laminated film in specified numbers and supplied to the consumer.

The consumer, i.e. the person performing the test, will at the time of using the test, immediately peel off the protection sheet 2, once he has soaked the small test material 3 of this detection implement with the saliva to be tested. He will then fold the adhesive sheet at the folding line 6 with the adhesive portion facing inwardly, thus causing the inner facets (areas "a" and "b" of 1) to adhere firmly to each other. At this point, the adhesive area which has become exposed because of the difference in the size of areas "a" and "b", will adhere to a heated body, which is heated to a temperature around 35°C. In a short period of 15 minutes or so the color of the test piece will change enabling the determination of the number of living bacteria. The only condition which the heated body, article or substance used to facilitate the reaction is that its temperature must be constant so as to enable the reaction of the cells of the living bacteria with the reagent. Good results are, for instance, obtainable by merely applying the implement directly to the human skin. Furthermore, as the test materials are held between adhesive sheets under such conditions as to be protected from the atmosphere and heated in such a way that the risk of the living bacteria being lost is eliminated, the reaction environment is kept constant and therefore reproduceable results are obtainable. Thus, the simplified detection implement of this invention will not require any special apparatus even in the case of group examination of school children. It is not only convenient and suitable to obtain the examination results in a short time, but also lends itself to production on an industrial scale.

As a heating apparatus or heated body, an incubator may be used, but a metal surface, glass surface, a container with warm water heated by a wolfram filament bulb, an infrared lamp or even the surface of the skin of human beings could serve the same purpose.

Example 1

The following is an example in which the detection implement of this invention is utilized as a dental caries activity testing apparatus for the diagnosis of caries.

EP-A-0,097,904 discloses a simple discrimination method of dental caries and its implement.

The gist of this prior art is that, a sample liquid, for instance a given amount of saliva containing cariogenous microorganisms is applied dropwise onto a dried small test material soaked with a certain reagent ingredient liquid. It is pushed together and covered tightly at the central portions of two transparent adhesive sheets. The material is then stored and kept at a constant temperature of 37°C for at least 15 minutes. The amount of cariogenous microorganisms contained in the saliva correlates with the degree of color change of the small test material. This is a simple method and device used to determine the stage of a cariogenous process.

In this previous implement, the components of the reagents were 0.05 to 0.003 weight % of resazurin, triphenyl tetrazorium, neotetrasorium, 2,6-dichlorophenol indophenol or methylorange or their salts and 10 weight % of sucrose.

The reagent components used in the example of the present invention were resazurin 0.05% and sucrose 10%. A hard and round filtration paper having a thickness of 0.8 mm and a diameter of 8 mm was soaked with this solution, then dried and used as the small test material.

The protection sheet was a brown oval 50 mm x 110 mm shaped kraft paper and the surface to come into contact with the adhesive material was coated with a silicone resin. An opening of 9 mm in diameter was cut at the spot corresponding approximately to the center of area "a" of the adhesive sheet. The adhesive sheet measured 45 mm in diameter at the "a" area and 30 mm at the "b" area. The adhesive sheet and protection sheet were adhered tightly so that the approximate center of area "a" of the adhesive sheet tallied with the opening in the protection sheet. The small test material prepared above was affixed to the surface of the adhesion material (9 mm in diameter) of the adhesive sheet which is exposed by the opening in the protection sheet. Furthermore, the adhesive sheet 1 was made from polyvinyl chloride and had a thickness of 20µm. The area "a" in Fig. 1B was painted totally white and area "b" was transparent.

The person performing the test will collect one drop of human saliva and allow it to drip onto the small test material 3. He will then immediately peel off the protection sheet 2 and fold the "b" area (Fig. 1B) of the adhesive sheet at the folding line 6 so that the adhesive material becomes the inside and the small test material is covered by the "b" area of the adhesive sheet, whereby the atmosphere is removed as much as possible. Once area "b" adheres tightly to area "a", a horseshoe or inversed "U" shaped adhesive facet about 7 mm wide will be exposed around the "a" area (Fig. 2). This adhesive facet should be immediately placed and affixed onto a heated body, e.g the inner part of the upper arm of a human being. Having been in place for about 15 minutes, the small test material will undergo color changes. From the degree of color change, a rough estimation of the number of bacteria causing the caries can be made.

In other words, this invention has the advantages that the implement is simple in structure easy to produce and moreover lends itself to mass production. As a result, the implement can be supplied at low cost. Furthermore, the implement is small due to its flat board-like shape and can be affixed to a heated body or heating apparatus. When the heated body or heating apparatus is the human or animal skin, the implements can be applied to it safely and easily like a plaster. As the reacting substance is a laminated material wrapped up inside a film no environmental pollution is caused and the implement is later reduced to ashes.

The advantages mentioned above will reduce the examination costs for group examination where many people are examined in a short time. The usefulness of the implement is tremendous.

Example 2
Another embodiment of the detection implement of this invention was prepared according to Example 1, except that the adhesive sheet 1 was shaped as a square (Fig. 4) and serially linked (Fig. 5).

Example 3
Another embodiment of the detection implement of this invention was prepared in the same manner as in Example 1, except that the adhesive sheet and protection sheet were given a round (Fig. 6) and square form (Fig. 7). The size was the same and the sheets were serially linked (Fig. 8).

Example 4
Another embodiment of the detection implement of this invention was prepared in the same manner as in Example 1, except that the areas "a" and "b" of the adhesive sheet 1 were cut off separately and area "a" was given a square and area "b" a round shape (Figs. 9A and 9B).

The detection implement of this invention differs from that disclosed in EP-A 0,097,904 in that an opening is made in the protection sheet in advance, and the adhesive surface is exposed through this opening, into which the small test material is placed. Area "b" is cut off separately from the same sheet as "a", but as area "b" is intended to cover the small test material 3, it does not necessarily have to be a film with an adhesive surface.

Example 5
Another embodiment of the detection implement of this invention is prepared. The shape of the adhesive sheet was made simpler than that of the various examples mentioned above. The detection implements in Drawings 10A and 10B are much simpler in shape. The adhesive sheet is a film strip cut around at both ends. Consequently, there will be less cutting waste and production will thus be more economical. As the adhesion surface to be stuck to a heated body or heating apparatus forms a crescent or a finger nail shape at the rounded rim of "a", there will be no adhesive area around the small test material (three facets) unlike the implements in the other drawings. Even when a simple heated body is used, some sort of a cover is put over said test material to render heating more efficient. Thus, the desired purpose can be attained with one adhesion surface only as shown in this drawing.

Example 6

The basic embodiment of the detection implement of this invention, is the implement shown in Figs. 11A, 11B and 11C prepared in a manner analogous to that of Example 1. The "a" and "b" areas of the transparent sheet will form a round shape when laid one upon another.

Example 7

In this example, the protection sheet "2" possesses a break portion (or break portions) in order to facilitate the separation or peeling off of the protection sheet "2" with fingers or finger nails.

Claims

1. A detection implement which comprises
a) an adhesive sheet (1) having an adhesion material layer (4) as well as an area "a" of a round, square or any other optimal shapes and an area "b" of a round, square or any other optimal shape with a folding line (6) at the border of areas "a" and "b";
b) a small test material (3) adhered to the adhesion material layer (4) and placed approximately at the center of area "a" of the adhesive sheet (1); and
c) a peelable protection sheet (2) covering the entire surface of the adhesion material layer (4) and having an opening (5) big enough to expose the whole surface of the small test material (3), wherein after removal of the protection sheet (2) and overlapping the areas "a" and "b" by folding the adhesive sheet (1) at the folding line (6) the small test material (3) is covered and a part of area "a" of the adhesive sheet (1) is exposed to act as the adhesive portion for attachment to a heating apparatus or a heated body.

2. A detection implement according to claim 1 in which the adhesive sheet (1) is colorless and transparent or colored and transparent or opaque.

3. A detection implement according to claim 1 or 2 in which the small test material (3) is of a porous inorganic or organic substance.

4. A detection implement according to Claim 1 or 2 in which the small test material (3) is a small test paper that contains or does not contain reagents for detection in advance.

5. A detection implement according to any one of the Claims 1, 2, and 3 in which a plurality of the small test material (3), adhesion material layer (4) and the transparent adhesive sheet (1) form a stack layer and also serially linked on the protection sheet (2).

6. A detection implement according to any of Claims 1 to 5 in which the protection sheet (2) is colorless semi-transparent or opaque or colored semi-transparent or opaque.

7. A detection implement according to any one of the preceding claims, in which the areas "a" and "b" of the adhesive sheet are cut off separately.

8. A detection implement according to any one of the preceding claims, in which the protection sheet possesses a break portion or break portions thereon that make ease to peel off said protection sheet.

9. A detection implement according to any one of the preceding claims, in which the area "b" possesses no adhesion material layer when area "b" being smaller than area "a".

**Patentansprüche**

1. Prüfvorrichtung mit:
a) einem adhäsiven Blatt (1) mit einer Klebstoffschicht (4) aus einer Fläche "a" mit runder, quadratischer oder beliebiger anderer optimaler Form sowie einer Fläche "b" mit runder, quadratischer oder beliebiger anderer optimaler Form mit einer Faltlinie (6) an der Grenze der Flächen "a" und "b";
b) einem kleinen Testmaterial (3), das an der Klebstoffschicht (4) anhaftet und ungefähr im Zentrum der Fläche "a" des adhäsiven Blattes (1) angeordnet ist; und
c) einer abziehbaren Schutzschicht (2), die die gesamte Oberfläche der Klebstoffschicht (4) bedeckt, und eine Öffnung (5) aufweist, die groß genug ist, um die gesamte Oberfläche des kleinen Testmaterials (3) freizulegen,
wobei nach Entfernen der Schutzschicht (2) und Überlappen der Flächen "a" und "b" durch Falten des adhäsiven Blattes (1) an der Faltlinie (6) das kleine Testmaterial (3) bedeckt und ein Teil der Fläche "a" des adhäsiven Blattes (1) freigelegt ist, um als Klebabschnitt zur Befestigung an einem Heizgerät oder einem erwärmten Körper zu wirken.

2. Prüfvorrichtung nach Anspruch 1, wobei das adhäsive Blatt (1) farblos und durchsichtig oder farbig und durchsichtig oder undurchsichtig ist.

3. Prüfvorrichtung nach Anspruch 1 oder 2, wobei das kleine Testmaterial (3) aus einer porösen anorganischen oder organischen Substanz besteht.

4. Prüfvorrichtung nach Anspruch 1 oder 2, wobei das kleine Testmaterial (3) ein kleines Testpapier ist, das Reagenzien zur Vorprüfung enthält oder nicht enthält.

5. Prüfvorrichtung nach einem der Ansprüche 1 bis 3, wobei das kleine Testmaterial (3), die Klebstoffschicht (4) und das durchsichtige adhäsive Blatt (1) einen Schichtstapel ausbilden und auch nacheinander mit der Schutzschicht (2) verbunden sind.

6. Prüfvorrichtung nach einem der Ansprüche 1 bis 5, wobei die Schutzschicht (2) farblos halbdurchsichtig oder undurchsichtig oder gefärbt halbdurchsichtig oder undurchsichtig ist.

7. Prüfvorrichtung nach einem der vorangegangenen Ansprüche, wobei die Flächen "a" und "b" des adhäsiven Blatts in getrennte Stücke geschnitten sind.

8. Prüfvorrichtung nach einem der vorangegangenen Ansprüche, wobei die Schutzschicht eine Unterbrechung oder Unterbrechungen darin aufweist, die das Abziehen der Schutzschicht erleichtern.

9. Prüfvorrichtung nach einem der vorangegangenen Ansprüche, wobei die Fläche "b" keine Klebstoffschicht aufweist, wenn die Fläche "b" kleiner als die Fläche "a" ist.

**Revendication**

1. Un dispositif de détection qui comprend:

(a) une feuille d'adhésif (1) ayant une couche de matériau d'adhérence (4) ainsi qu'une zone "a" de forme ronde, carrée ou de toute autre forme optimale et une zone "b" de forme ronde, carrée ou de toute autre forme optimale avec une ligne de pliage (6) en bordure des zones "a" et "b";

(b) un petit matériau d'essai (3) qui adhère à la couche de matériau d'adhérence (4) et qui est placé approximativement au centre de la zone "a" de la feuille d'adhésif (1); et

(c) une feuille de protection arrachable (2) recouvrant la surface entière de la couche de matériau d'adhérence (4) et ayant une ouverture (5) suffisamment grande pour exposer la surface totale du petit matériau d'essai (3),

de façon qu'après enlèvement de la feuille de protection (2) et juxtaposition des zones "a" et "b" par pliage de la feuille d'adhésif (1) au niveau de la ligne de pliage (6), le petit matériau d'essai (3) est recouvert et une partie de la zone "a" de la feuille d'adhésif (1) est exposée pour agir comme une portion adhésive pour la fixation à un appareil de chauffage ou à un corps chauffé.

2. Un dispositif de détection selon la revendication 1 selon lequel la feuille d'adhésif (1) est incolore et transparente ou colorée et transparente ou opaque.

3. Un dispositif de détection selon la revendication 1 ou 2 selon lequel le petit matériau d'essai (3) est une substance poreuse et inorganique ou organique.

4. Un dispositif de détection selon la revendication 1 ou 2 selon lequel le petit matériau d'essai (3) est un petit morceau de papier d'essai qui contient ou ne contient pas à l'avance des réactifs pour la détection.

5. Un dispositif de détection selon l'une quelconque des revendications 1, 2 et 3 selon lequel une pluralité de petits matériaux d'essai (3), de couches de matériaux d'adhérence (4) et de feuilles d'adhésifs transparentes (1) forment une couche empilée et également liée en série à la feuille de protection (2).

6. Un dispositif de détection selon l'une quelconque des revendications 1 à 5 selon lequel la feuille de protection (2) est incolore, semi-transparente ou opaque ou colorée, semi-transparente ou opaque.

7. Un dispositif de détection selon l'une quelconque des revendications précédentes, selon lequel les zones "a" et "b" de la feuille d'adhésif sont découpées séparément.

8. Un dispositif de détection selon l'une quelconque des revendications précédentes, selon lequel la feuille de protection présente une portion ou des portions interrompues pour faciliter l'arrachement de ladite feuille de protection.

9. Un dispositif de détection selon l'une quelconque des revendications précédentes, selon lequel la zone "b" ne possède aucune couche de matériau d'adhérence lorsque la zone "b" est plus petite que la zone "a".

# FIG. 1A

# FIG. 1B

# FIG. 2

# FIG. 3

1

# FIG. 4

# FIG. 5

2

# FIG. 6

# FIG. 7

# FIG. 8

# FIG. 9A

# FIG. 9B

# FIG. 10A

# FIG. 10B

# FIG. 11A

# FIG. 11B

# FIG. 11C